# EUROPÄISCHE PATENTANMELDUNG

(11) **EP 3 799 066 A1**
(43) Veröffentlichungstag der Anmeldung: **31.03.2021**
(21) Anmeldenummer: 19199226.2
(22) Anmeldetag: 24.09.2019
(51) Int. Cl.: G16H 40/63

(54) **SCHULEN VON NUTZERN MEDIZINISCHER GERÄTE**

(71) Anmelder: Siemens Healthcare GmbH, 91052 Erlangen (DE)
(72) Erfinder: Blum, Thomas, 91077 Neunkirchen A. Br (DE)

(57) **Zusammenfassung**

Verfahren (S1-S8), bei dem eine Bedienung eines medizinischen Geräts (1-4) durch einen Nutzer automatisch auf verbesserungsfähige Bedienhandlungen überwacht wird (S1) und, falls mindestens eine verbesserungsfähige Bedienhandlung erkannt wird (S2), dem Nutzer automatisch mindestens eine zugehörige Abhilfemaßnahme angeboten wird (S5). Ein System (1-4) weist ein medizinisches Gerät (1-3) und mindestens ein Nachschulungsmodul (4) auf, wobei das Nachschulungsmodul (4) dazu eingerichtet ist, das Verfahren ablaufen zu lassen. Die Erfindung ist insbesondere anwendbar auf die Schulung von Nutzern eines Magnetresonanztomographie (MR)-Geräts, eines Computertomographie (CT)-Geräts, eines Geräts zur molekularen Bildgebung ("Molecular Imaging"), eines röntgenbasierten Angiographie-Geräts, usw.

## Beschreibung

Die Erfindung betrifft ein Verfahren, insbesondere zum Schulen von Nutzern medizinischer Geräte. Die Erfindung betrifft auch ein System, das ein medizinisches Gerät aufweist. Die Erfindung betrifft ferner ein Computerprogrammprodukt und ein computerlesbares Speichermedium, die Befehle aufweisen, welche bei ihrer Ausführung durch eine Datenverarbeitungseinrichtung diese veranlassen, das Verfahren durchzuführen. Die Erfindung ist insbesondere anwendbar auf die Schulung von Nutzern eines Magnetresonanztomographie (MR)-Geräts, eines Computertomographie (CT)-Geräts, eines Geräts zur molekularen Bildgebung ("Molecular Imaging"), eines röntgenbasierten Angiographie-Geräts, usw.

Zur effizienten Bedienung und Erzeugen qualitativ hochwertiger Ergebnisse wird bei modernen Medizingeräten ein hohes Maß an Wissen des Bedieners oder Nutzers vorausgesetzt.

Bisher wird dieses Wissen im Rahmen von vorgefertigten, oft mehrtägigen Schulungsmaßnahmen in Form von Einzel- oder Gruppenschulungen und/oder durch Schulungsunterlagen im Selbststudium vermittelt. Dabei können diese Maßnahmen, abhängig vom Vorwissen des Schulungsteilnehmers, bei komplexen Systemen schnell mehrere Tage oder Wochen in Anspruch nehmen. Diese Schulungsmaßnahmen sind in dem Sinne statisch, dass es zwar unterschiedliche Schulungen für unterschiedliche Zielgruppen gibt, die Teilnehmerzahl jedoch begrenzt ist, was dazu führt, dass Schulungsteilnehmer entweder mit Themen konfrontiert werden, die schon bekannt oder für ihre aktuelle Arbeit nicht relevant sind, oder für bestimmte Schulungsteilnehmer relevante Themen überhaupt nicht angesprochen werden.

Aus Zeit- und Kostengründen werden auch oft nicht alle als Nutzer oder Bediener vorgesehenen Mitarbeiter geschult, sondern es wird sich darauf verlassen, dass eine Weitergabe des Wissens und der Schulungsunterlagen unter den Mitarbeitern erfolgt. Dadurch kann es zu fehlendem Wissen über neue Funktionalitäten des medizinischen Geräts, Fehlbedienungen, zahlreichen Rückfragen beim Hersteller (Helpdesk) und/oder zu zeit- und/oder kostenintensiven On-Site-Schulungen kommen.

Es ist die Aufgabe der vorliegenden Erfindung, die Nachteile des Standes der Technik zumindest teilweise zu überwinden und insbesondere eine technisch einfach umsetzbare Möglichkeit zur Schulung, insbesondere Nachschulung, von Benutzern bzw. Bedienpersonal medizinischer Geräte bereitzustellen.

Diese Aufgabe wird gemäß den Merkmalen der unabhängigen Ansprüche gelöst. Bevorzugte Ausführungsformen sind insbesondere den abhängigen Ansprüchen entnehmbar.

Die Aufgabe wird gelöst durch ein Verfahren, bei dem eine Bedienung eines medizinischen Geräts durch einen Nutzer automatisch auf verbesserungsfähige Bedienhandlungen überwacht wird und, falls mindestens eine verbesserungsfähige Bedienhandlung erkannt wird, dem Nutzer automatisch mindestens eine zugehörige Abhilfemaßnahme angeboten wird.

Dieses Verfahren ergibt den Vorteil, dass Abhilfemaßnahmen (die auch als Schulungsmaßnahmen bezeichnet werden können) auf den tatsächlich vorhandenen, während des Betriebs des Systems erkannten oder ermittelten Schulungsbedarfs des Nutzers individuell abstimmbar sind. Unnötige Abhilfe- oder Schulungsmaßnahmen werden vermieden, was deren Effizienz und Effektivität verbessert. Ferner wird vorteilhafterweise eine Motivation eines Nutzers, die eigene Vorgehensweise zu verbessern, verstärkt. Darüber hinaus erhöht sich die Qualität der durch das medizinische Gerät erlangten Ergebnisse, insbesondere Untersuchungsergebnisse. Desweiteren wird eine Zahl von Rückfragen bei dem Hersteller oder Händler des Geräts verringert. Ein weiterer Vorteil besteht darin, dass Ergebnisse der Überwachung der Systembedienung und Schulungsmaßnahmen zum Verbessern einer Bedienerfreundlichkeit ("Usability") durch den Hersteller verwendet werden können.

Das medizinische Gerät kann insbesondere ein diagnostisches Gerät sein. Das medizinische Gerät kann beispielsweise ein MR-Gerät, ein CT-Gerät, ein Gerät zur molekularen Bildgebung, ein röntgenbasiertes Angiographie-Gerät, usw. sein. Das Gerät kann ein eigenständiges Gerät bzw. Einzelgerät oder ein medizinisches System aus mehreren, insbesondere datentechnisch miteinander verbundenen Komponenten sein. Beispielsweise kann das Gerät ein MR-System mit einem MR-Scanner und einer Bedienstation sein. Eine Bildgebungseinrichtung kann eine weitere Komponente des MR-Systems sein oder in eine vorhandene Komponente integriert sein, z.B. in die Bedienstation.

Unter einer Bedienhandlung kann insbesondere jede durch das Gerät erfassbare Handlung, Eingabe, Handhabung usw. verstanden werden. Insbesondere kann darunter jede an einer Nutzerschnittstelle des Geräts vorgenommene Handlung verstanden werden, z.B. eine Eingabe von Daten, ein Aufrufen bestimmter Eingabemasken, usw.

Das eine Bedienhandlung automatisch überwacht wird, umfasst insbesondere, dass dies mittels eines automatisierten Ablaufs ohne Zwischenschaltung menschlicher Überwachung geschieht. Unter einem Überwachen kann insbesondere ein Analysieren verstanden werden. Die Überwachung wird für bestimmte, individuell identifizierbare Nutzer vorgenommen. Die Identifizierung kann z.B. in Form einer Personalnummer, Nutzerkennung usw. vorliegen und ermöglicht eine eindeutige Verknüpfung eines bestimmten Nutzers mit zugehörigen bisherigen oder potenziell anbietbaren Schulungsmaßnahmen.

Der Nutzer kann auch als Bediener bezeichnet werden. Das Verfahren kann beispielsweise als Verfahren zum Schulen von Nutzern medizinischer Geräte, Verfahren zum Betreiben eines medizinischen Geräts, Verfahren zum Steigern einer Bedieneffizienz eines medizinischen Geräts o.ä. bezeichnet werden.

Unter einer "verbesserungsfähigen Bedienhandlung" wird insbesondere eine Bedienhandlung verstanden, welche nicht innerhalb eines vorgegebenen "normalen" Nutzerverhaltens liegt. Das "normale" Nutzerverhalten umfasst insbesondere eine innerhalb vorgegebener zeitlicher Grenzen korrekt ausgeführte Bedienhandlung oder Folge von Bedienhandlungen. Die Bedienhandlungen sind insbesondere Abschnitte eines Workflows zur Gerätebedienung.

Es ist eine Ausgestaltung, dass die mindestens eine verbesserungsfähige Bedienhandlung mindestens eine Handlung aus der folgenden Gruppe umfasst:
- eine Fehleingabe,
- eine Fehlkonfiguration,
- einen Abbruch einer Eingabe,
- eine korrekte, aber verzögerte Bedienhandlung,
- eine korrekte, aber verzögerte Folge von Bedienhandlungen,
- Aufruf einer zu der Bedienhandlung gehörigen Hilfefunktion.

Diese Bedienhandlungen weisen den Vorteil auf, dass sie zuverlässig auf ein zu verbesserndes Wissensfeld eines Nutzers hinweisen, was wiederum eine Auswahl einer geeigneten Abhilfemaßname erleichtert. Die verbesserungsfähige Bedienhandlung kann insbesondere an einer Nutzerschnittstelle des Geräts vorgenommen worden sein, z.B. an einer Bedienkonsole oder einem Nutzerarbeitsplatz.

Unter einer verzögerten Bedienhandlung wird insbesondere eine Bedienhandlung verstanden, die nicht innerhalb eines vorgegebenen Zeitrahmens abgeschlossen worden ist. Analog wird unter einer verzögerten Folge von Bedienhandlung insbesondere eine Folge von Bedienhandlung verstanden, die nicht innerhalb eines vorgegebenen Zeitrahmens abgeschlossen worden ist.

Es ist eine Ausgestaltung, dass die mindestens eine zugehörige Abhilfemaßnahme mindestens eine Maßnahme oder Handlung bzw. Aktion aus der folgenden Gruppe umfasst:
- Anbieten eines Durchführens einer die verbesserungsfähige Bedienhandlung betreffenden Schulungsaktivität zum Selbststudium an dem Gerät, beispielsweise als interaktive Schulungsaktivität;
- Anbieten einer Bediensimulation, welche eine verbesserungsfähige Bedienhandlung oder Folge von Bedienhandlungen umfasst, an dem Gerät;
- Aussenden einer Benachrichtigung an eine externe Instanz betreffend den Nutzer und die verbesserungsfähige Bedienhandlung oder Folge von Bedienhandlungen, falls die Bedienhandlung mehrfach als verbesserungsfähig erkannt worden ist.

Diese Abhilfemaßnahmen weisen den Vorteil auf, dass sie zeitnah zu der erkannten verbesserungsfähigen Bedienhandlung (oder Folge von Bedienhandlungen) und speziell darauf zugeschnitten angeboten werden können, so dass ein Nutzer eine hohe Motivation besitzt, sie durchzuführen.

Die Schulungsaktivität zum Selbststudium kann ein Dokument, ein Video, eine Online-Schulung usw. sein.

Die Bediensimulation kann anhand des Geräts beruhend auf korrekten Bedienhandlungen durchgeführt werden.

Die externe Instanz kann ein Anbieter zugehöriger Abhilfe- oder Schulungsmaßnahmen und/oder ein Hersteller oder Händler (Distributor) des Geräts sein. Im ersten Fall können dem Nutzer vorteilhafterweise auf seine Wissensdefizite zugeschnittene Schulungsmaßnahmen angeboten werden. Im zweiten Fall kann der Hersteller oder Händler vorteilhafterweise Verbesserungen an dem Gerät vornehmen, z.B. durch eine verbesserte Benutzerführung.

Es ist eine Ausgestaltung, dass die mindestens eine angebotene Abhilfemaßnahme zusätzlich beruhend auf zuvor korrekt und verzögerungsfrei durchgeführten Bedienhandlungen angepasst wird. Dadurch wird der Vorteil erreicht, dass ein Nutzer besonders zuverlässig nur Abhilfemaßnahmen erhält, die auf die verbesserungsfähigen Bedienhandlungen gerichtet sind und von diesem Nutzer zuvor korrekt und verzögerungsfrei durchgeführte Bedienhandlungen nicht oder nur kurzzeitig umfasst sind. So wird eine für den Nutzer redundante Abhilfe vermieden, was wiederum einen Lernerfolg steigert.

Es ist eine Ausgestaltung, dass die mindestens eine angebotene Abhilfemaßnahme zusätzlich beruhend auf Ergebnissen von zuvor durchgeführten Nutzerschulungen angepasst wird. So wird der Vorteil erreicht, dass Abhilfemaßnamen mit den zuvor erreichten Ergebnissen kombiniert werden können, beispielsweise um hartnäckigen oder sich langfristig haltenden Wissensdefiziten des Nutzers priorisiert mit Abhilfemaßnahmen zu begegnen.

Es ist eine Ausgestaltung, dass dann, wenn mindestens eine zugehörige Abhilfemaßnahme durch den Nutzer angenommen wird oder worden ist, diese mindestens eine Abhilfemaßnahme an dem Gerät durchgeführt wird. So kann vorteilhafterweise eine besonders praxisnahe und zeitnahe Abhilfemaßnahme bereitgestellt werden. Beispielsweise kann die Abhilfemaßnahme wie ein Video oder ein Dokument an einem Bildschirm des Geräts angezeigt werden, beispielsweise parallel oder gleichzeitig zur einer realen Bedienung des Geräts.

Es ist eine Ausgestaltung, dass mindestes eine Abhilfemaßname an einer Nutzerschnittstelle des Geräts durchgeführt wird. So kann vorteilhafterweise eine ganz besonders praxisnahe und zeitnahe Abhilfemaßnahme bereitgestellt werden.

Die Auswahl oder Anpassung einer Abhilfe- oder Schulungsmaßnahmen kann insbesondere in folgender Weise erfolgen:
- Ein medizinisches Gerät (Einzelgerät oder medizinisches System) hält einen Satz von insbesondere kurzen Abhilfemaßnahmen, z.B. als Text oder Video, zum Selbststudium vor. Auf der Basis der Ergebnisse der Überwachung oder Analyse der Bedienhandlungen und/oder bisherigen Abhilfemaßnahmen werden nach Erkennen einer verbesserungsfähigen Bedienhandlung daraus geeignete Abhilfe- oder Schulungsmaßnahmen ausgewählt und dem Nutzer direkt an dem Gerät zur Ausführung angeboten. Abhilfemaßnahmen, die Bereiche betreffen, in denen der Nutzer keine Probleme bei der Bedienung des Geräts gezeigt hat, werden nicht angeboten.
- Ein medizinisches Gerät stellt alternativ oder zusätzlich die Möglichkeit bereit, komplexe Anwendungsfälle oder "Use Cases" (z.B. die Registrierung eines neuen Patienten oder die Durchführung einer Patientenuntersuchung) direkt an einer Nutzerschnittstelle (beispielsweise einer Bedienkonsole) des Geräts zu simulieren und den Nutzer durch die notwendigen Bedienschritte zu führen. Fehlbedienungen und Fehlkonfigurationen werden dabei sofort erkennbar, was den Lernerfolg steigert.
- Werden beim Durchführen der auf den Nutzer zugeschnittenen oder adaptierten Abhilfemaßnahmen bzw. beim Bedienen des Geräts durch das Gerät wiederholt Probleme wie oben beschrieben beobachtet oder erkannt, kann eine automatische Information an eine zuständige Schulungsorganisation erfolgen, z.B. um weitere, persönliche Schulungsmaßnahmen zu diskutieren.
- Die Ergebnisse der Überwachung oder Analyse einer Gerätebedienung zusammen mit den Ergebnissen von Abhilfemaßnahmen können dem Nutzer an der Nutzerschnittstelle des Geräts, z.B. einem Bedienmonitor visualisiert werden, um ihm die Möglichkeit zu eröffnen, den eigenen Lernfortschritt zu beobachten.

Die Aufgabe wird auch gelöst durch ein System, aufweisend ein medizinisches Gerät und mindestens ein Nachschulungsmodul, wobei das Nachschulungsmodul dazu eingerichtet ist,
- eine Bedienung des Geräts durch einen Nutzer automatisch auf verbesserungsfähige Bedienhandlungen zu überwachen und,
- falls mindestens eine verbesserungsfähige Bedienhandlung erkannt wird, dem Nutzer automatisch mindestens eine zugehörige Abhilfemaßnahme anzubieten.

Das System kann analog zu dem Verfahren ausgebildet werden und ergibt die gleichen Vorteile.

Es ist eine Ausgestaltung, dass das Nachschulungsmodul ein geräteexternes Modul ist, das mit dem medizinischen Gerät datentechnisch verbunden ist. Beispielsweise kann das Nachschulungsmodul eine eigenständige Vorrichtung sein, z.B. ein mit dem medizinischen Gerät datentechnisch verbundener Rechner, der zur Durchführung des Verfahrens eingerichtet ist, z.B. programmiert ist. Der Rechner kann beispielsweise ein Netzwerkserver oder ein Cloudrechner sein.

Es ist eine Ausgestaltung, dass das Nachschulungsmodul in das Gerät integriert ist, z.B. ein Modul des medizinischen Geräts im engeren Sinne ist. Das Nachschulungsmodul kann auch rein funktional in dem Gerät implementiert sein, z.B. durch eine entsprechende Programmierung des medizinischen Geräts, insbesondere ohne dedizierte oder zusätzliche Hardware.

Die Aufgabe wird auch gelöst durch ein Computerprogrammprodukt, das Befehle aufweist, die bei der Ausführung des Programms durch eine Datenverarbeitungseinrichtung, insbesondere das oben beschriebene Modul, diese veranlassen, das Verfahren wie oben beschrieben durchzuführen. Das Computerprogrammprodukt kann analog zu dem Verfahren ausgebildet sein und ergibt die gleichen Vorteile. Das Computerprogrammprodukt kann beispielsweise als Web-Applikation vorliegen, die auf das medizinische Gerät oder ein eigenständiges Nachschulungsmittel heruntergeladen werden kann.

Die Aufgabe wird auch gelöst durch ein computerlesbares Speichermedium, das Befehle aufweist, die bei ihrer Ausführung durch eine Datenverarbeitungseinrichtung diese veranlassen, das Verfahren wie oben beschrieben durchzuführen.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden schematischen Beschreibung eines Ausführungsbeispiels, die im Zusammenhang mit den Zeichnungen näher erläutert werden. Dabei können zur Übersichtlichkeit gleiche oder gleichwirkende Elemente mit gleichen Bezugszeichen versehen sein.

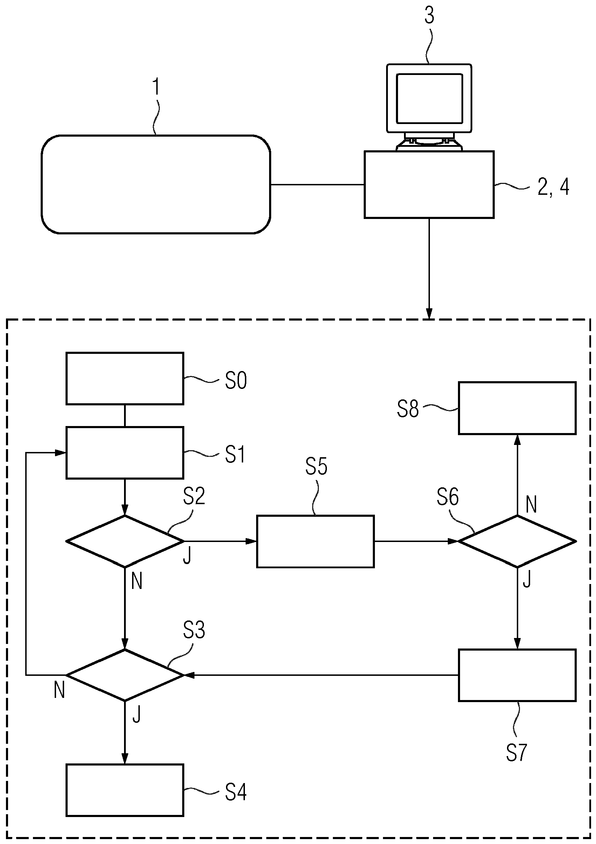

Fig.1 zeigt eine Skizze eines medizinischen Geräts in Form eines MR-Systems 1 bis 4 und ein darauf ablaufbares Verfahren mit den Verfahrensschritten S1 bis S8. Das MR-System 1 bis 4 umfasst hier beispielsweise einen MR-Scanner 1 und eine Bedienstation 2 mit einer Nutzerschnittstelle mit einem Bedienmonitor 3. Die Bedienstation 2 kann dazu eingerichtet sein, mit dem MR-Scanner aufgenommene Messdaten für eine MR-Bildgebung zu verwenden. In die Bedienstation 2 ist durch entsprechende Programmierung ein Nachschulungsmodul 4 integriert.

Die Bedienstation 2 ist durch das Nachschulungsmodul 4 dazu eingerichtet, zunächst in einem Schritt S0 einen Nutzer zur Bedienung des MR-Systems 1 bis 4 zu registrieren (z.B. über eine den Nutzer individuell kennzeichnende Registrierungsnummer, Personalnummer, Login-ID, usw.) und einen durchzuführenden Workflow aufzurufen.

In einem Schritt S1 wird durch den Nutzer eine Bedienhandlung oder Folge von Bedienhandlungen des Workflows vorgenommen bzw. durch das Nachschulungsmodul 4 erkannt.

In einem Schritt S2 wird mittels des Nachschulungsmoduls 4 analysiert, ob die in Schritt S1 vorgenommene Bedienhandlung oder Folge von Bedienhandlungen verbesserungsfähig ist oder nicht. Eine verbesserungsfähige Bedienhandlung kann beispielsweise vorliegen, wenn in Schritt S1
- eine Fehleingabe und/oder eine Fehlkonfiguration vorgenommen wurde;
- eine Eingabe abgebrochen wurde;
- eine Bedienhandlung oder Folge von Bedienhandlungen korrekt, aber verzögert vorgenommen wurde bzw. die Bedienhandlung zu lange dauerte; und/oder
- eine zu einer Bedienhandlung gehörige Hilfefunktion aufgerufen wurde.

Liegt keine verbesserungsfähige Bedienhandlung vor ("N"), wird in einem Schritt S3 überprüft, ob der Workflow abgearbeitet worden ist.

Ist dies der Fall ("J"), wird das Verfahren in einem Schritt S4 beendet. Schritt S4 kann umfassen, dass die im Laufe des Verfahrens aufgetretenen verbesserungswürdigen Bedienhandlungen und ggf. auch die nicht verbesserungswürdigen Bedienhandlungen mit dem bestimmten Nutzer verknüpft gespeichert werden, z.B. um daraus weitere Schulungsmaßnahmen abzuleiten und/oder um sie für folgende Abhilfe- oder Schulungsmaßnahmen zu berücksichtigen.

Ist in Schritt S3 festgestellt worden, dass der Workflow noch nicht abgearbeitet worden ist ("N"), wird zu Schritt S1 zurückverzweigt.

Wird in Schritt S2 hingegen festgestellt, dass mindestens eine verbesserungsfähige Bedienhandlung vorliegt ("J"), wird in einem Schritt S5 dem Nutzer automatisch mindestens eine zugehörige Abhilfemaßnahme angeboten.

In einem Schritt S6 wird eine Entscheidung des Nutzers abgefragt, ob dieser die Abhilfemaßname sofort durchführen möchte ("J") oder nicht ("N").

Falls ja, wird in einem Schritt S7 die Abhilfemaßnahme durchgeführt. Dies kann beispielsweise umfassen, dass eine die verbesserungsfähige Bedienhandlung betreffende Schulungsaktivität zum Selbststudium dargestellt oder abgespielt wird, insbesondere an dem MR-System 1 bis 4, speziell an dem Bedienmonitor 3. Auch kann eine Bediensimulation, welche die verbesserungsfähige Bedienhandlung umfasst, durchgeführt werden, insbesondere an dem MR-System 1 bis 4, speziell an der Nutzerschnittstelle mit dem Bedienmonitor 3. Ferner kann einer Benachrichtigung an eine externe Instanz betreffend den Nutzer und die verbesserungsfähige Bedienhandlung ausgesandt werden, insbesondere falls die Bedienhandlung mehrfach als verbesserungsfähig erkannt worden ist.

Führt die Abfrage in Schritt S6 jedoch zu der Entscheidung, dass die Abhilfemaßname nicht sofort durchgeführt werden soll ("N"), kann sie ignoriert werden oder in einem Schritt S8 zur späteren Ausführung vorgehalten werden. Nach Schritt S8 wird zu Schritt S3 verzweigt (o. Abb.).

Obwohl die Erfindung im Detail durch das gezeigte Ausführungsbeispiel näher illustriert und beschrieben wurde, so ist die Erfindung nicht darauf eingeschränkt und andere Variationen können vom Fachmann hieraus abgeleitet werden, ohne den Schutzumfang der Erfindung zu verlassen.

Allgemein kann unter "ein", "eine" usw. eine Einzahl oder eine Mehrzahl verstanden werden, insbesondere im Sinne von "mindestens ein" oder "ein oder mehrere" usw., solange dies nicht explizit ausgeschlossen ist, z.B. durch den Ausdruck "genau ein" usw.

## Patentansprüche

1. Verfahren (S1-S8), bei dem
- eine Bedienung eines medizinischen Geräts (1-4) durch einen Nutzer automatisch auf verbesserungsfähige Bedienhandlungen überwacht wird (S1) und,
- falls mindestens eine verbesserungsfähige Bedienhandlung erkannt wird (S2), dem Nutzer automatisch mindestens eine zugehörige Abhilfemaßnahme angeboten wird (S5).

2. Verfahren (S1-S8) nach Anspruch 1, bei dem die mindestens eine verbesserungsfähige Bedienhandlung mindestens eine Bedienhandlung aus der folgenden Gruppe umfasst:
- eine Fehleingabe,
- eine Fehlkonfiguration,
- einen Abbruch einer Eingabe,
- eine korrekte, aber verzögerte Bedienhandlung,
- eine korrekte, aber verzögerte Folge von Bedienhandlungen,
- Aufruf einer zu der Bedienhandlung gehörigen Hilfefunktion.

3. Verfahren (S1-S8) nach einem der vorhergehenden Ansprüche, bei dem die mindestens eine zugehörige Abhilfemaßnahme mindestens eine Abhilfemaßnahme aus der folgenden Gruppe umfasst:
- Anbieten eines Durchführens einer die verbesserungsfähige Bedienhandlung betreffenden Schulungsaktivität zum Selbststudium an dem Gerät;
- Anbieten einer Bediensimulation, welche die verbesserungsfähige Bedienhandlung umfasst, an dem Gerät;
- Aussenden einer Benachrichtigung an eine externe Instanz betreffend den Nutzer und die verbesserungsfähige Bedienhandlung, falls die Bedienhandlung mehrfach als verbesserungsfähig erkannt worden ist.

4. Verfahren (S1-S8) nach einem der vorhergehenden Ansprüche, bei dem die mindestens eine angebotene Abhilfemaßnahme zusätzlich beruhend auf zuvor korrekt und verzögerungsfrei durchgeführten Bedienhandlungen angepasst wird.

5. Verfahren (S1-S8) nach einem der vorhergehenden Ansprüche, bei dem die mindestens eine angebotene Abhilfemaßnahme beruhend auf Ergebnissen von zuvor durchgeführten Nutzerschulungen angepasst wird.

6. Verfahren (S1-S8) nach einem der vorhergehenden Ansprüche, bei dem, falls mindestens eine zugehörige Abhilfemaßnahme angenommen wird (S6), diese mindestens eine Abhilfemaßnahme an dem Gerät durchgeführt wird (S7).

7. Verfahren (S1-S8) nach Anspruch 6, bei dem mindestes eine Abhilfemaßname an einer Nutzerschnittstelle (3) des Geräts (1-4) durchgeführt wird.

8. System (1-4), aufweisend ein medizinisches Gerät (1-3) und mindestens ein Nachschulungsmodul (4), wobei das Nachschulungsmodul (4) dazu eingerichtet ist,
- eine Bedienung des Geräts durch einen Nutzer automatisch auf verbesserungsfähige Bedienhandlungen zu überwachen und,
- falls mindestens eine verbesserungsfähige Bedienhandlung erkannt wird, dem Nutzer automatisch mindestens eine zugehörige Abhilfemaßnahme anzubieten.

9. System (1-4) nach Anspruch 8, bei dem das Nachschulungsmodul ein geräteexternes Modul ist.

10. System (1-4) nach einem der Ansprüche 8 oder 9, bei dem das Nachschulungsmodul (4) in das Gerät (1-3) integriert ist.

11. Computerprogrammprodukt, aufweisend Befehle, die bei der Ausführung des Programms durch eine Datenverarbeitungseinrichtung diese veranlassen, das Verfahren (S1-S8) nach einem der Ansprüche 1 bis 7 durchzuführen.

12. Computerlesbares Speichermedium, aufweisend Befehle, die bei ihrer Ausführung durch eine Datenverarbeitungseinrichtung diese veranlassen, das Verfahren (S1-S8) nach einem der Ansprüche 1 bis 7 durchzuführen.
